# EUROPEAN PATENT SPECIFICATION

(11) **EP 1 546 103 B1**
(45) Date of publication and mention of the grant of the patent: **18.03.2009**
(21) Application number: 03756899.5
(22) Date of filing: 02.10.2003
(51) Int. Cl.: C07D 211/60, C07D 207/16, A61K 31/496, A61P 25/28

(54) **PIPERAZINE AND PIPERIDINE DERIVATIVES FOR TREATMENT OF NEUROLOGICAL DISEASES**
PIPERAZIN UND PIPERADIN-DERIVATE FÜR DIE BEHANDLUNG VON NEUROLOGISCHEN KRANKHEITEN
PIPERAZINE ET DERIVES DE PIPERIDINE PERMETTANT DE TRAITER DES MALADIES NEUROLOGIQUES

(30) Priority: 03.10.2002 US 416134 P
(43) Date of publication of application: 29.06.2005
(73) Proprietor: VERTEX PHARMACEUTICALS INCORPORATED, Cambridge, MA 02139 (US)
(72) Inventor: LAUFFER, David, J., Stow, MA 01775 (US); BOTFIELD, Martyn, C., Boston, MA 02115 (US); ECKARD, Ottow, Moltkestrasse 48 (DE)
(74) Representative: Burrichter, Arwed Andreas
(86) International application number: PCT/US2003/031080
(87) International publication number: WO 2004/031148

(56) References cited:
- WO-A-00/10533
- WO-A-00/32588
- WO-A-01/58891
- WO-A-98/20893
- US-A- 5 811 434
- US-A- 6 043 255
- K. MAGYAR ET. AL.: "The action of trelibet, a new antidepressive agent on 3H-Noradrenaline release fron rabbit pulmonary artery." EUROPEAN JOURNAL OF PHARMACOLOGY, vol. 130, 1986, pages 219-27, XP009024386

## Description

### TECHNICAL FIELD OF THE INVENTION

The present invention relates to piperazine and piperidine derivatives, which are especially useful for treating or preventing neuronal damage, particularly damage associated with neurological diseases. These compounds are also useful for stimulating nerve growth. The invention also provides compositions comprising the compounds of the present invention and the use of there compounds in the manufacture of a medicament for treating or preventing neuronal damage or for stimulating nerve growth.

### BACKGROUND OF THE INVENTION

Neurological diseases are associated with the death of or injury to neuronal cells. Typical treatment of neurological diseases involves drugs capable of inhibiting neuronal cell death. A more recent approach involves the promotion of nerve regeneration by promoting neuronal growth.

Neuronal growth, which is critical for the survival of neurons, is stimulated *in vitro* by nerve growth factors (NGF). For example, Glial Cell Line-Derived Neurotrophic Factor (GDNF) demonstrates neurotrophic activity both, *in vivo* and in vitro, and is currently being investigated for the treatment of Parkinson's disease. Insulin and insulin-like growth factors have been shown to stimulate growth of neurites in rat pheochromocytoma PC12 cells and in cultured sympathetic and sensory neurons [Recio-Pinto et al., J. Neurosci., 6, pp. 1211-1219 (1986)]. Insulin and insulin-like growth factors also stimulate the regeneration of injured motor nerves in vivo and in vitro [Near et al., Proc. Natl. Acad. Sci., pp. 89, 11716-11720 (1992); and Edbladh et al., Brain Res., 641, pp. 76-82 (1994)]. Similarly, fibroblast growth factor (FGF) stimulates neural proliferation [D. Gospodarowicz et al., Cell Differ., 19, p. 1 (1986)] and growth [M. A. Walter et al., Lymphokine Cytokine Res., 12, p. 135 (1993)].

There are, however, several disadvantages associated with the use of nerve growth factors for treating neurological diseases. They do not readily cross the blood-brain barrier. They are unstable in plasma and they have poor drug delivery properties.

Recently, small molecules have been shown to stimulate neurite outgrowth *in vivo*. In individuals suffering from a neurological disease, this stimulation of neuronal growth protects neurons from further degeneration, and accelerates the regeneration of nerve cells. For example, estrogen has been shown to promote the growth of axons and dendrites, which are neurites sent out by nerve cells to communicate with each other in a developing or injured adult brain [(C. Dominique Toran-Allerand et al., J. Steroid Biochem. Mol. Biol., 56, pp. 169-78 (1996); and B. S. McEwen et al., Brain Res. Dev. Brain. Res., 87, pp. 91-95 (1995)]. The progress of Alzheimer's disease is slowed in women who take estrogen. Estrogen is hypothesized to complement NGF and other neurotrophins and thereby help neurons differentiate and survive.

Other target sites for the treatment of neurodegenerative disease are the immunophilin class of proteins. Immunophilins are a family of soluble proteins that mediate the actions of immunosuppressant drugs such as cyclosporin A, FK506 and rapamycin. Of particular interest is the 12 kDa immunophilin, FK-506 binding protein (FKBP12). FKBP12 binds FK-506 and rapamycin, leading to an inhibition of T-cell activation and proliferation. Interestingly, the mechanism of action of FK-506 and rapamycin are different. For a review, see, S. H. Solomon et al., Nature Med.., 1, pp. 32-37 (1995). It has been reported that compounds with an affinity for FKBP12 that inhibit that protein's rotomase activity possess nerve growth stimulatory activity. [Lyons et al., Proc. Natl. Acad. Sci. USA, 91, pp. 3191-3195 (1994)]. Many of these such compounds also have immunosuppressive activity.

FK506 (Tacrolimus) has been demonstrated to act synergistically with NGF in stimulating neurite outgrowth in PC12 cells as well as sensory ganglia [Lyons et al. (1994)]. This compound has also been shown to be neuroprotective in focal cerebral ischemia [J. Sharkey and S. P. Butcher, Nature, 371, pp. 336-339 (1994)] and to increase the rate of axonal regeneration in injured sciatic nerves [B. Gold et al., J. Neurosci., 15, pp. 7509-16 (1995)].

The use of immunosuppressive compounds, however, has drawbacks in that prolonged treatment with these compounds can cause nephrotoxicity [Kopp et al., J. Am. Soc. Nephrol., 1, p. 162 (1991)], neurological deficits [P.C. DeGroen et al., N. Eng. J. Med., 317, p. 861 (1987)] and vascular hypertension [Kahan et al., N. Eng. J. Med., 321, p. 1725 (1989)].

Sub-classes of FKBP binding compounds which inhibit rotomase activity, but which purportedly lack immunosuppressive function have been disclosed for use in stimulating nerve growth and for neuroprotection [see, United States patent 5,614,547; WO 96/40633; WO 96/40140; WO 97/16190; WO 98/13343; WO 98/13355; WO 98/29116; WO 98/29117; WO 98/35675; WO 98/37882; WO 98/37885; J. P. Steiner et al., Proc. Natl. Acad. Sci. USA , 94, pp. 2019-23 (1997); and G. S. Hamilton et al., Bioorg. Med. Chem. Lett., 7, pp. 1785-90 (1997)].

Stimulation of neural axons in nerve cells by piperidine derivatives is described in WO 96/41609. Clinical use of the piperidine and pyrrolidine derivatives known so far for stimulating axonal growth has not been promising, as the compounds are unstable in plasma and do not pass the blood-brain barrier in adequate amounts.

More recently, classes of compounds which lack the ability to bind FKBP and lack immunosuppressive function have been described for use in stimulating nerve growth and preventing neurodegeneration [see, WO 98/20891; WO 98/20892; WO 98/20893 and WO 99/10340. WO 01/58891 A2 describes piperazine and piparidine derivatives, which are useful for treating or preventing neuronal damage.

Though a wide variety of compounds for treating or preventing neurological degenerative diseases have been described, only two of these are currently in clinical trials and none have been approved for commercialization. And while compounds which share certain structural similarities to the compounds disclosed herein have been described in United States patent Nos. 4,115,569 and 4,374,990, neither of those patents specifically teach or suggest the compounds of the present invention, nor is there any teaching that such compounds would have utility in stimulating nerve growth or preventing neurodegeneration.

Thus, there remains a need for the discovery and design of new compounds and compositions that have the ability to prevent and/or treat neuronal damage associated with neuropathologic conditions.

### SUMMARY OF THE INVENTION

The present invention provides a compound having formula IA: 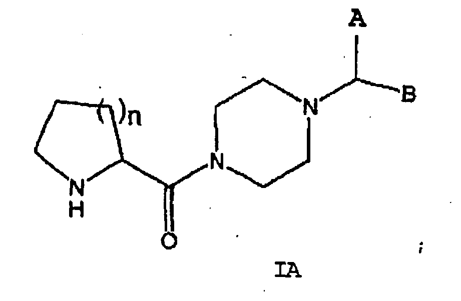 wherein:
n is 1 or 2;
A and B each is independently selected from phenyl, chlorophenyl, dichlorophenyl, fluorophenyl, or difluorophenyl provided that if n is 2 A, B are not simultaneously phenyl and that the compound having formula IA is not

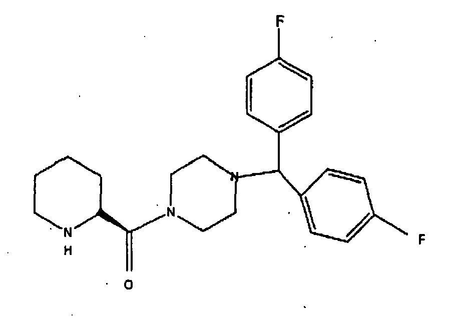

In another embodiment, the invention provides pharmaceutical compositions comprising the compounds of formula IA. These compositions may be utilized in methods for promoting neuronal repair or preventing neuronal damage in an *ex vivo* nerve cell. More particularly, the compounds of this invention are useful in the manufacture of a medicament for treating various neurological diseases. Examples of such diseases include peripheral nerve destruction due to physical injury or diseases such as diabetes; physical injuries to the central nervous system (e.g., brain or spinal cord); stroke; neurological disturbances due to nerve degeneration, such as Parkinson's disease, Alzheimer's disease, and amylotrophic lateral sclerosis.

### DETAILED DESCRIPTION OF THE INVENTION

The present invention provides a compound having formula IA: 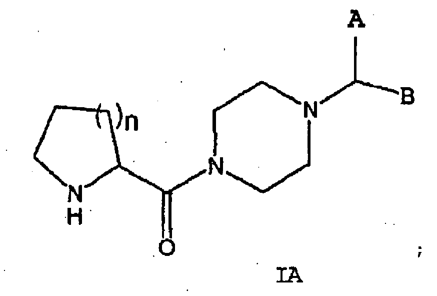 wherein:
n is 1 or 2;
A and B each is independently selected from phenyl, chlorophenyl, dichlorophenyl, fluorophenyl, or difluorophenyl provided that if n is 2 A, B are not simultaneously phenyl and that the compound having formula IA is not

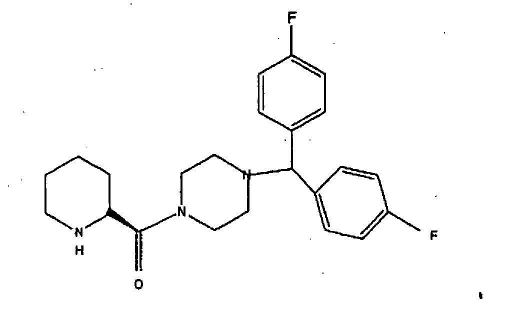

Preferably, n is 1. According to another preferred embodiment, n is 2.

Most preferably, the compounds of the present invention have the following formulae:

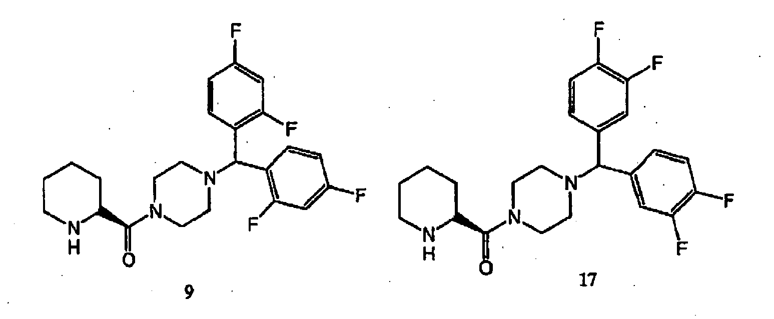

The compounds of formula IA may be stereoisomers,

geometric isomers or stable tautomers. The invention envisions all possible isomers, such as E and Z isomers, S and R enantiomers, diastereoisomers, racemates, and mixtures of those.

The compounds of the present invention may be readily prepared using known synthetic methods. For example, compounds of the formula I 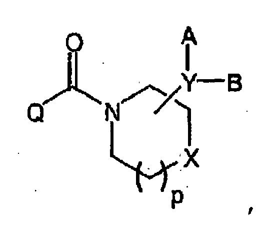 wherein:
each Q is a 3-7 membered monocyclic saturated or partially unsaturated ring having 1-4 heteroatoms selected from N, O or S;
wherein up to 4 hydrogen atoms in Q are optionally and independently replaced with halo, -OH, =O, =N-OR¹, (C₁-C₆)-straight or branched alkyl, Ar-substituted-(C₁-C₆)-straight or branched alkyl, (C₂-C₆)-straight or branched alkenyl or alkynyl, Ar-substituted-(C₂-C₆)-straight or branched alkenyl or alkynyl, O-(C₁-C₆)-straight or branched alkyl, O-[(C₁-C₆)-straight or branched alkyl]-Ar, O-(C₂-C₆)-straight or branched alkenyl or alkynyl, O-[(C₂-C₆)-straight or branched alkenyl or alkynyl]-Ar, or O-Ar;
wherein Q has at least one NH ring atom group;
each R¹ is independently selected from (C₁-C₆)-straight or branched alkyl, Ar-substituted-(C₁-C₆)-straight or branched alkyl, cycloalkyl-subatituted-(C₁-C₆)-straight or branched alkyl, (C₂-C₆)-straight or branched alkenyl or alkynyl, or Ar-substituted-(C₂-C₆)-straight or branched alkenyl or alkynyl; wherein
one to two CH₂ groups of said alkyl, alkenyl, or alkynyl chains in R¹ are optionally and independently replaced with O, S, S(O), S(O)₂, C(O) or N(R²), wherein when R¹ is bound to nitrogen, the CH₂ group of R¹ bound directly to said nitrogen cannot be replaced with C(O);
Ar is selected from phenyl, 1-naphthyl, 2-naphthyl, indenyl, azulenyl, 2-furyl, 3-furyl, 2-thienyl, 3-thienyl, 2-pyridyl, 3-pyridyl, 4-pyridyl, pyrrolyl, oxazolyl, thiazolyl, imidazolyl, pyraxolyl, pyrazolinyl, pyraolidinyl, isoxazolyl, isothiazolyl, 1,2,3-oxadiazolyl, 1,2,3-triazolyl, 1,3,4-thiadiazolyl, 1,2,4-triazolyl, 1,2,4-oxadiazolyl, 1,2,4-thiadiazolyl, 1,2,3-thiadiazolyl, benoxazolyl, pyridazinyl, 2-pyrimidinyl, 4-pyrimidinyl, 5-pyrimidinyl, pyrazinyl, 1,3,5-triazinyl, 1,3,5-trithianyl, indolizinyl, indolyl, isoindolyl, 3H-indolyl, indolinyl, benzo[b]furanyl, benzo[b]thiophenyl, 1H-indazolyl, benzimidazolyl, benzthiazolyl, purinyl, 4H-quinolizinyl, quinolinyl, 1,2,3,4-tetrahydroisoquinolinyl, isoquinolinyl, 1,2,3,4-tetrahydroquinolinyl, cinnolinyl, phthalazinyl, quinazolinyl, quinoxalinyl, 1,8-naphthyridinyl, or any other chemically feasible monocyclic or bicyclic ring system, wherein each ring consists of 5 to 7 ring atoms and wherein each ring comprises 0 to 3 heteroatoms independently selected from N, O, or S, wherein
each Ar is optionally and independently substituted with one to three substituents selected from halo, hydroxy, nitro, -SO₃H, =O, trifluoromethyl, trifluoromethoxy, (C₁-C₆)-straight or branched alkyl, (C₁-C₆)-straight or branched alkenyl, O-[(C₁-C₆)-straight or branched alkyl], O-[(C₁-C₆)-straight or branched alkenyl], O-benzyl, 0-phenyl, 1,2-methylenedioxy, -(R³)(R⁴), carboxyl, N-(C₁-C₆-straight or branched alkyl or C₂-C₆-straight or branched alkenyl) carboxamides, N,N-di-(C₁-C₆-straight or branched alkyl or C₂-C₆-straight or branched alkenyl) carboxamides, N-(C₁-C₆-straight or branched alkyl or C₂-C₆-straight or branched alkenyl) sulfonamides, or N,N-di-(C₁-C₆-straight or branched alkyl or C2-C6-straight or branched alkenyl) sulfonamides;
each of R³ and R⁴ are independently selected from (C₁-C₆)-straight or branched alkyl, (C₂-C₆)-straight or branched alkenyl or alkynyl, hydrogen, phenyl or benzyl; or wherein R³ and R⁴ are taken together with the nitrogen atom to which they are bound to form a 5-7 membered heterocyclic ring;
each R² is independently selected from hydrogen, (C₁-C₆)-straight or branched alkyl, or (C₂-C₆)-straight or branched alkenyl or alkynyl;
X is selected from C(R²)₂, N, N(R²), O, S, S(O), or S(O)₂
Y is selected from a bond, -O-, (C₁-C₆)-straight or branched) alkyl, or (C₂-C₆)-straight or branched) alkenyl or alkynyl; wherein Y is bonded to the depicted ring via a single bond or a double bond; and wherein one to two of the CH₂ groups of said alkyl, alkenyl, or alkynyl is optionally and independently replaced with O, S, S(O), S(O)₂, C(O) or N(R);
p is 0, 1 or 2;
each of A and B is independently selected from hydrogen or Ar; or one of A or B is absent; and
wherein two carbon ring atoms in the depicted ring structure may be linked to one another via a C₁-C₄ straight alkyl or a C₂-C₄ straight alkenyl to create a bicyclic moiety,
   may be prepared as shown below in scheme 1:

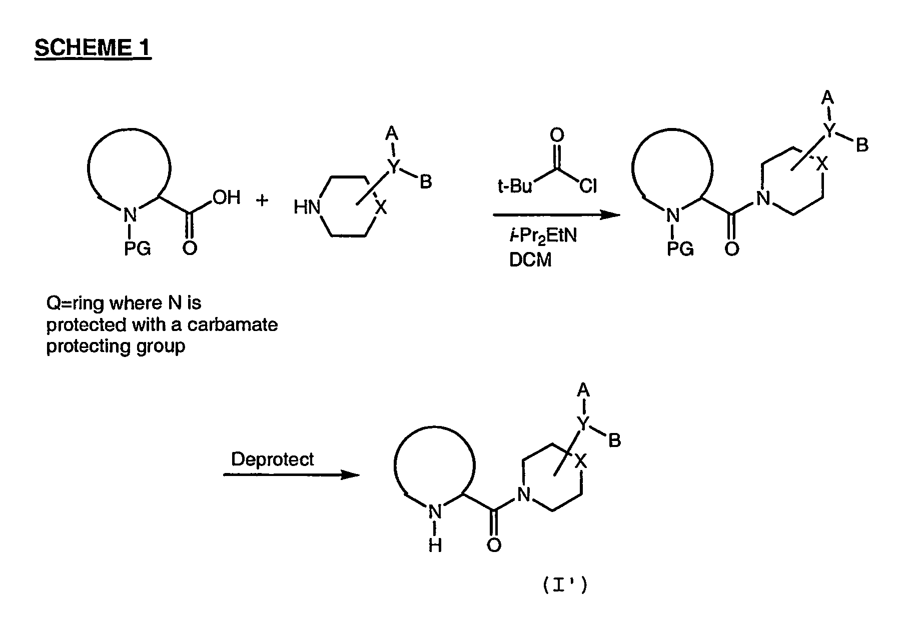

In the scheme depicted above, the following
abbreviations are used: iPr₂EtN = diisopropylethylamine;
DCM = dichloromethane;

One of skill in the art will be well aware of analogous synthetic methods for preparing other compounds of formula (I).

The nerve growth stimulatory activity of the compounds of this invention may be initially assayed using several cell culture assays known in the art. For example, the compounds of this invention may be tested in a neurite outgrowth assay using pheochromocytoma PC12 cells as described by Lyons et al., PNAS, 91, pp. 3191-3195 (1994). A similar assay may be carried out in SH-SY5Y human neuroblastoma cells. Alternatively, the chick dorsal root ganglia assay described in United States patent 5,614,547 or in G. S. Hamilton et al., Bioorg. Med. Chem. Lett., (1997) and references cited therein, may be utilized.

The compounds of this invention may also be assayed for nerve growth stimulatory activity in vivo using a mouse model of Parkinson's disease [J. P. Steiner et al., Proc. Natl. Acad. Sci. USA, 94, pp. 2019-23 (1997), United States patent 5,721,256] or following surgical sciatic nerve crush in rats.

The neuroprotective activity of the compounds of this invention may be assayed using rat embryo ventral mesencephalic cells in culture which are subsequently exposed to the glutamate receptor agonist NMDA. This assay is described in detail in the example section.

According to another embodiment, this invention provides compositions comprising a compound of formula IA and a pharmaceutically acceptable carrier.

Pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxy methylcellulose, polyacrylates, waxes, polyethylene-polyoxypropylene-block polymers, polyethylene glycol and wool fat.

In another embodiment, the pharmaceutical composition of the present invention is comprised of a compound of formula IA a pharmaceutically acceptable carrier, and one or more additional therapeutic agents.

For example, the additional agent can be a neurotrophic factor.

The term "neurotrophic factor," as used herein, refers to compounds which are capable of stimulating growth or proliferation of nervous tissue. Numerous neurotrophic factors have been identified in the art and any of those factors may be utilized in the compositions of this invention. These neurotrophic factors include, but are not limited to, nerve growth factor (NGF), insulin-like growth factor (IGF-1) and its active truncated derivatives such as gIGF-1 and Des(1-3)IGF-I, acidic and basic fibroblast growth factor (aFGF and bFGF, respectively), platelet-derived growth factors (PDGF), brain-derived neurotrophic factor (BDNF), ciliary neurotrophic factors (CNTF), glial cell line-derived neurotrophic factor (GDNF), neurotrophin-3 (NT-3)and neurotrophin 4/5 (NT-4/5). The most preferred neurotrophic factor in the compositions of this invention is NGF.

As used herein, the described compounds used in the pharmaceutical compositions and methods of this invention, are defined to include pharmaceutically acceptable derivatives thereof. A "pharmaceutically acceptable derivative" denotes any pharmaceutically acceptable salt, ester, or salt of such ester, of a compound of this invention or any other compound which, upon administration to a patient, is capable of providing (directly or indirectly) a compound of this invention, or a metabolite or residue thereof, characterized by the ability to promote repair or prevent damage of neurons from disease or physical trauma.

If pharmaceutically acceptable salts of the described compounds are used, those salts are preferably derived from inorganic or organic acids and bases. Included among such acid salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, 3-phenyl-propionate, picrate, pivalate, propionate, succinate, tartrate, thiocyanate, tosylate and undecanoate. Base salts include ammonium salts, alkali metal salts, such as sodium and potassium salts, alkaline earth metal salts, such as calcium and magnesium salts, salts with organic bases, such as dicyclohexylamine salts, N-methyl-D-glucamine, and salts with amino acids such as arginine, lysine, and so forth. Also, the basic nitrogen-containing groups can be quaternized with such agents as lower alkyl halides, such as methyl, ethyl, propyl, and butyl chloride, bromides and iodides; dialkyl sulfates, such as dimethyl, diethyl, dibutyl and diamyl sulfates, long chain halides such as decyl, lauryl, myristyl and stearyl chlorides, bromides and iodides, aralkyl halides, such as benzyl and phenethyl bromides and others. Water or oil-soluble or dispersible products are thereby obtained.

The described compounds utilized in the compositions and methods of this invention may also be modified by appending appropriate functionalities to enhance selective biological properties. Such modifications are known in the art and include those which increase biological penetration into a given biological system (e.g., blood, lymphatic system, central nervous system), increase oral availability, increase solubility to allow administration by injection, alter metabolism and alter rate of excretion.

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously.

Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as Ph. Helv or similar alcohol.

The pharmaceutical compositions of this invention may be orally administered in any orally acceptable dosage form including, but not limited to, capsules, tablets, aqueous suspensions or solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added.

Alternatively, the pharmaceutical compositions of this invention may be administered in the form of suppositories for rectal administration. These can be prepared by mixing the agent with a suitable non-irritating excipient which is solid at room temperature but liquid at rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The amount of both a described compound and the optional neurotrophic factor that may be combined with the carrier materials to produce a single dosage form will vary depending upon the host treated and the particular mode of administration. Preferably, the compositions should be formulated so that a dosage of between 0.01 - 100 mg/kg body weight/day of the described compound can be administered. If a neurotrophic factor is present in the composition, then a dosage of between 0.01 µg - 100 mg/kg body weight/day of the neurotrophic factor can be administered to a patient receiving these compositions.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of active ingredients will also depend upon the particular described compound and neurotrophic factor in the composition.

According to another embodiment, this invention provides methods for promoting repair or preventing neuronal damage in an *ex vivo* nerve cell. Such methods comprise the step of treating nerve cells, glial cells, chromafin cells or stem cells with any of the compounds described above. Preferably, this method promotes repair or prevents neuronal damage and the compound is formulated into a composition additionally comprising a pharmaceutically acceptable carrier. The amount of the compound utilized in these methods is between about 0.01 and 100 mg/kg body weight/day.

According to an alternate embodiment, the method of promoting repair or preventing neuronal damage comprises the additional step of treating nerve cells with a neurotrophic factor, such as those contained in the pharmaceutical compositions of this invention. This embodiment includes administering the compound and the neurotrophic agent in a single dosage form or in separate, multiple dosage forms. If separate dosage forms are utilized, they may be administered concurrently, consecutively or within less than about 5 hours of one another.

According to another embodiment, the methods of this

invention are used to stimulate axonal growth in nerve cells. The compounds are, therefore, suitable for treating or preventing neuronal damage caused by a wide variety of diseases or physical traumas. These include, but are not limited to, Alzheimer's disease, Parkinson's disease, ALS, Huntington's disease, Tourette's syndrome, multiple sclerosis, stroke and ischemia associated with stroke, neural paropathy, other neural degenerative diseases, motor neuron diseases, peripheral neuropathies including chemoneuropathies, sciatic injury, spinal cord or brain injuries, facial nerve damage, nerve damage associated with surgery or chemotherapy, retinopathy, macular degeneration, depression or schizophrenia

The methods of this invention used to stimulate axonal growth in nerve cells are also useful in increasing nerve graft survival and differentiation, increasing stem cell transplant survival and differentiation, and in increasing glial cell transplant survival and differentiation.

In a particularly preferred embodiment of the invention, the compounds are used in the manufacture of a medicament to treat a patient suffering from trigeminal neuralgia, glosspharyngeal neuralgia, Bell's Palsy, myasthenia gravis, muscular dystrophy, muscle injury, progressive muscular atrophy, progressive bulbar inherited muscular atrophy, herniated, ruptured, or prolapsed invertebrae disk syndrome's, cervical spondylosis, plexus disorders, thoracic outlet destruction syndromes, peripheral neuropathies, such as those caused by lead, dapsone, ticks, or porphyria, other peripheral myelin disorders, Alzheimer's disease, Gullain-Barre syndrome, Parkinson's disease and other Parkinsonian disorders, ALS, Tourette's syndrome, multiple sclerosis, other central myelin disorders, stroke and ischemia associated with stroke, neural paropathy, other neural degenerative diseases, motor neuron diseases, sciatic injury, neuropathy associated with diabetes, spinal cord injuries, facial nerve injury and other trauma, chemotherapy- and other medication-induced neuropathies, Huntington's disease, and protein fibrillization diseases, such as Diffuse Lewy Body disease, Alzheimer's disease-Lewy Body variant, Familla 1 British Dementia, and Frontotemporal Dementia.

More preferably, the compositions of the present invention are used for the manufacture of a medicament for treating Parkinson's disease, amylotrophic lateral sclerosis, Alzheimer's disease, stroke, neuralgias, muscular atrophies, and Guillain-Barre syndrome.

The above methods use compounds of formula IA.

More preferably, the above methods use compounds 9 and 17.

The medicaments prepared using the compounds according to the invention are administered in the form of a pharmaceutical preparation containing not only the active ingredient but also carriers, auxiliary substances, and/or additives suitable for enteric or parenteral administration. Administration can be oral or sublingual as a solid in the form of capsules or tablets, as a liquid in the form of solutions, suspensions, elixirs, aerosols or emulsions, or rectal in the form of suppositories, or in the form of solutions for injection which can be given subcutaneously, intramuscularly, or intravenously, or which can be given topically or intrathecally. Auxiliary substances for the desired medicinal formulation include the inert organic and inorganic carriers known to those skilled in the art, such as water, gelatin, gum arabic, lactose, starches, magnesium stearate, talc, vegetable oils, polyalkylene glycols, etc. The medicinal formulations may also contain preservatives, stabilizers, wetting agents, emulsifiers, or salts to change the osmotic pressure or as buffers.

Solutions or suspensions for injection are suitable for parenteral administration, and especially aqueous solutions of the active compounds in polyhydroxy-ethoxylated castor oil.

Surface-active auxiliary substances such as salts of gallic acid, animal or vegetable phospholipids, or mixtures of them, and liposomes or their components, can be used as carrier systems.

The neurotrophic effect of the compounds of formula (I) of the present invention and their physiologically acceptable salts can be determined using several cell culture assays known in the art or the assay described in Example 66. For example, the compounds of this invention may be tested in a neurite outgrowth using pheochromocytoma PC12 cells as described by W. E. Lyons et al., Proc. Natl. Acad. Sci. USA, 91, pp. 3191-3195 (1994). A similar assay may be carried out in SH-SY5Y human neuroblastoma cells. Alternatively, the chick dorsal root ganglia assay described in United States patent 5,614,547 or in G. S. Hamilton et al., Bioorg. Med. Chem. Lett., (1997) and references cited therein, may be utilized.

The compounds of this invention may also be assayed for nerve growth activity *in vivo* using a mouse model of Parkinson's disease [J. P. Steiner et al., Proc. Natl. Acad. Sci. USA, 94, pp. 2019-23 (1997)]

In order that this invention be more fully understood, the following examples are set forth. These examples are for the purpose of illustration only and are not to be construed as limiting the scope of the invention in any way.

Example 1

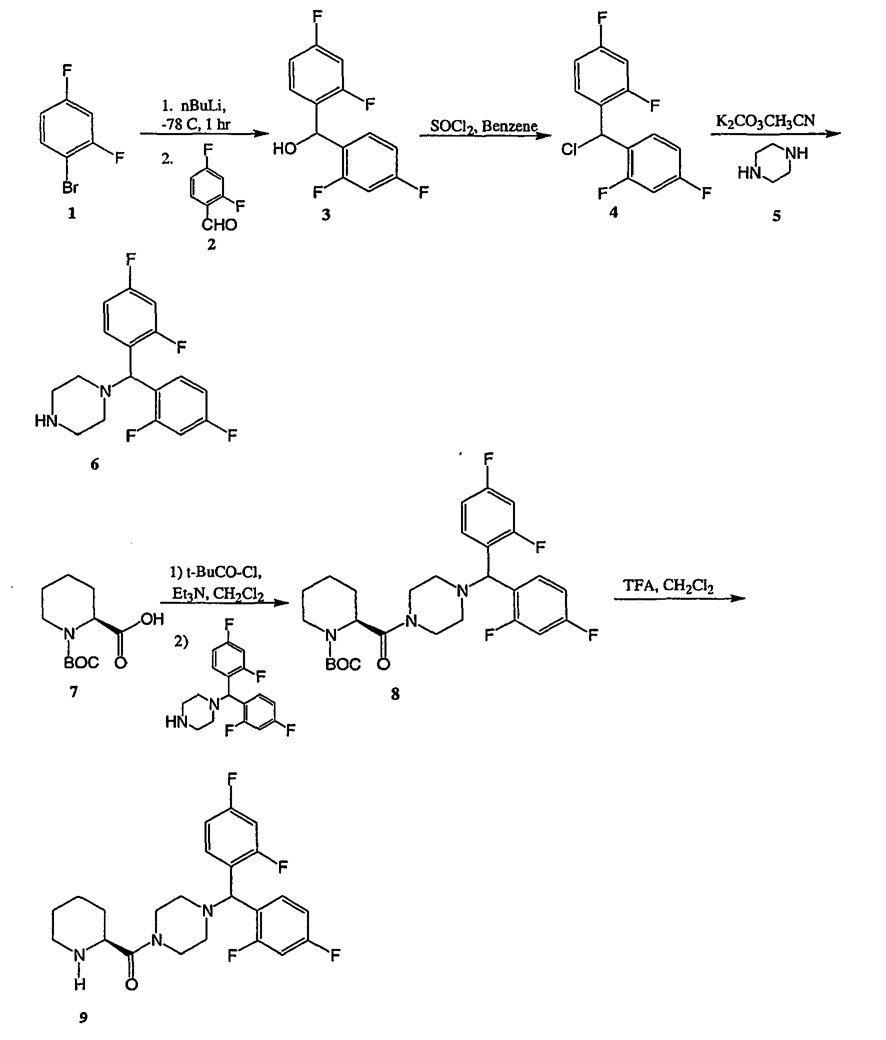

A) Bis-(2,4-difluoro-phenyl)-methanol 3

1-Bromo-2,4-difluoro-1-benzene (1, 201.18 g, 1.04 mol) was dissolved in anhydrous ether (1L). Butyl lithium (1.6M) (665 mL, 1.06 mol) was added at - 78 C over 60 minutes. After 2 hrs at - 78 C, 2,4-difluorobenzaldehyde (2, 146.65 g, 1.03 mol) was added dropwise to maintain the temperature below -65 C. The reaction mixture was allowed to warm up to room temperature overnight. After quenching the reaction with 1N HCl (600 mL), the organic phase was separated and the aqueous phase was extracted with ether (2 x 1L). The combined organic phases were washed with brine and dried over sodium sulfate then evaporated to dryness. The crude product 3 (quantitative yield) was used without further purification.

B) Bis-(2,4-difluorobenzhydryl) chloride **4**

To a solution of the crude **3** (273.37g, 1.07 mol), obtained as described above, in anhydrous benzene (750 mL), was added thionyl chloride (88.2 mL)., 1.21 mol). The reaction was refluxed and monitored by TLC; additional thionyl chloride (2 x 45 ml) was added after 30 mins. After 1 hr. under refluxed, the reaction was cooled to room temperature then evaporated under reduced pressure. The residue was azeotroped with two charges of heptanes and toluene to eliminate all traces of thionyl chloride. Crude **4** obtained was used immediately in the next step.

C) 1-[Bis-(2,4-difluoro-phenyl)-methyl]-piperazine **6**.

Crude **4** (243.9 g, 0.89 mol) was dissolved in acetonitrile (1550 ml) and piperazine **5** was added (765 g, 8.88 mol). Potassium carbonate (147.29g, 1.7 mol) was added under stirring, and the reaction was refluxed overnight. After cooling, mixture was filtered, and the filtrate was evaporated under reduced pressure. The crude was dissolved in ethyl acetate (2000 ml) then washed successively with water (5 x 500 ml) and brine (500 ml) and finally dried over sodium sulfate. The desired product **6** (237.66 g, 88%) was used onto the next step without further purification.

D) 2-{4-[Bis-(2,4-difluoro-phenyl)-methyl]piperazine-1-carbonyl}-piperidine-1-carboxylic acid tert-butyl ester **8**.

To a solution of (S)-(-)-1-(tert-butoxycarbonyl)-2-piperidine-carboxylic acid (**7**, 78.48 g, 0.34 mol) and triethylamine (95.4 ml, 0.68 mol) in methylene chloride (2280 ml) was added pivaloyl chloride (42.15 ml, 0.34 mol) dropwise at room temperature. After the addition was complete, the solution was stirred for 2 hrs. at room temperature then a solution of **6** (111.0 g, 0.34 mol) in methylene chloride (580 ml) was added over 1 hr. The reaction mixture was stirred overnight at room temperature, then washed with 10% NaOH (4 x 1L) and brine (2 x 1L). The organic layer was dried over sodium sulfate then evaporated under reduced pressure. The resulting dried yellow foam was dried in a high vacuum at room temperature to afford the pure product **8** (184.49 g) in 92% yield.

E) {4-[Bis-(2,4-difluoro-phenyl)-methyl]-piperazin-1-yl}-piperidin-2-yl-methanone **9**

To a solution of 2-{4-[Bis-(2,4-difluorophenyl)-methyl]piperazine-1-carbonyl}-piperidine-1-carboxylic acid tert-butyl ester **(8)** (13.14 g, 37.16 mmol) in methylene chloride (205 ml) was added dropwise trifluoroacetic acid (74 ml) at room temperature. The reaction mixture was stirred for 3 hrs. After the reaction was complete, volatiles were removed and the sample was concentrated in vacuo. The crude was dissolved in methylene chloride (100 ml) and washed with 1M NaOH (2 x 100 ml). The organic layer was dried over sodium sulfate then evaporated under reduced pressure to give 14.56 g of the compound 8 (90% yield) as a light yellow oil. The crude product was purified by chromatography on 340 g of silica gel (eluent: CH2C12/MeOH/ NH4OH; 95/5/0.1) to give the desired compound **9** as a white solid (8.67 g, 54% yield, mp= 84-85 °C).

MASS Spec: M+1 m/z = 436, M+2 m/z 437 (API-ES, positive mode). Optical Rotation: [α ]_{D}= -5.0° (c = 0.475 g/100 ml CHCl₃). HPLC (column: 50 mm C18) Rt 3.973 mins. (97.93% pure).

{4-[Bis-(2,4-difluoro-phenyl)-methyl]-piperazin-1-yl}-piperidin-2-yl-methanone dihydrochloride, **9.**

The compound **9** (5.59 g, 12.8 mmol) was dissolved in 20 ml of methylene chloride and diluted with 200 ml of diethyl ether. An anhydrous solution of 1.0 M HCl in diethyl ether (70 ml, 70 mmol) was added dropwise. A precipitate formed and the after stirring for 1.5 hours, the precipitate was collected and dried under vacuum at 50 °C to give the dihydrochloride salt, 6.06g. HPLC (C18 column (150 mm)): Rt 4.784 mins. (100% pure).

Example 2

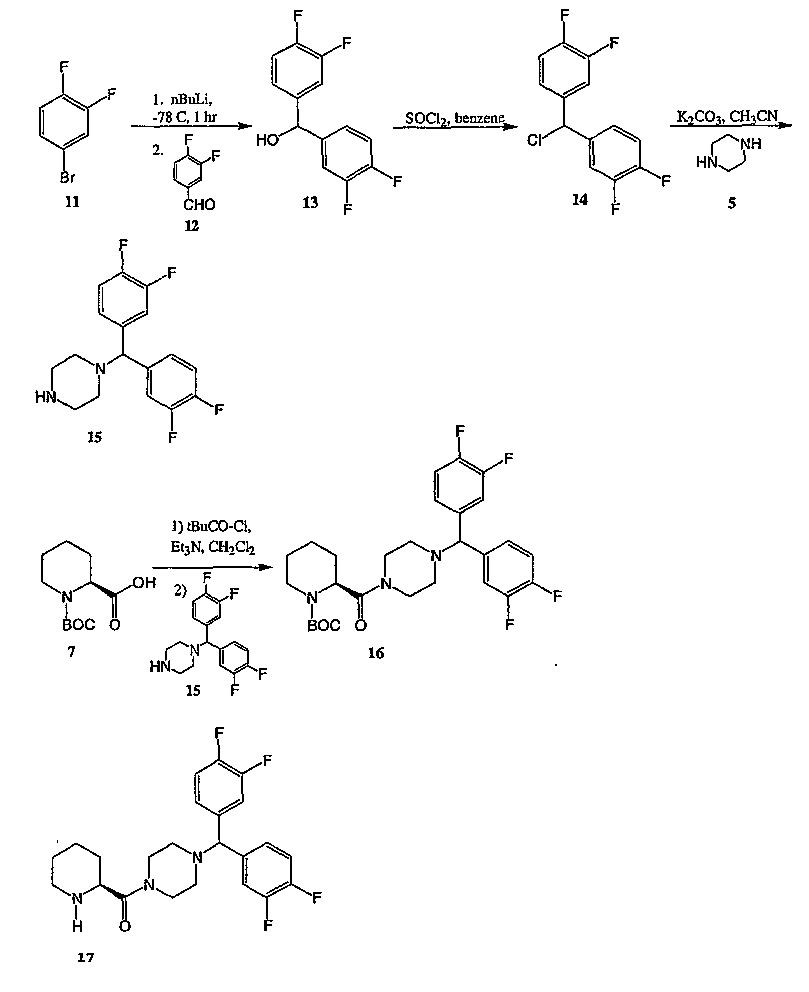

A) Bis-(3,4-difluoro-phenyl)-methanol **13.**

3,4-Difluoro-1-bromobenzene **11** (200 g, 1.04 mol) was dissolved in dry diethyl ether (1000 ml). A solution of n-butyl lithium (1.6M in hexane) (660 ml, 1.06 mol, 1.2 eq) was added at -78 C over 1 hr. under nitrogen. The reaction mixture was stirred at -78 C for another 2 hrs. and then 3,4-difluorobenzaldehyde **12** (146 g, 1.0 eq.) was added dropwise with the temperature kept below -70 C. The reaction mixture was stired at -70 C for 3 hrs. The reaction was warmed slowly to room temperature overnight. 1N HCl (500 ml) was added to quench the reaction. The organic phase was separated and the aqueous phase was extracted with diethyl ether (2 x 600ml). The combined organic phase was washed with brine (2 x 500 ml) and dried over sodium sulfate. After removal of solvent, 258 g (98%) of crude **13** was obtained as a brown oil. This crude product was used for the next reaction without further purification.

B) Bis-(3,4-difluorobenzhydryl) chloride **14.**

Thionyl chloride (136.8 g, 1.15 mol, 1.15 eq.) was added dropwise to a solution of **13** (258 g, 1 mol) in dry benzene (750 ml). The reaction was refluxed and monitored by TLC. After 2 hrs., additional thionyl chloride (68 g, 0.57 mol) was added. After another 1hr at reflux, the reaction was colled then evaporated under reduced pressure. Two charges of heptane and toluene were used to azeoptropically remove remaining traces of thionyl chloride, providing 268 g (97%) of crude **14** as a brown oil.

C) 1-(Bis-(3,4-difluorophenyl)-methyl)-piperazine **15.**

Crude **14** (248 g, 0.9 mol) was dissolved in acetonitrile (1400 ml) and piperazine 5 (752.6 g, 8.7 mol, 9.7 eq) was added. Potassium carbonate (145 g, 1.15 mol, 1.2 eq) was added with stirring, and the reaction was refluxed overnight. After cooling, the mixture was filtered and the filtrate was evaporated under pressure. The residue was dissolved in methylene chloride (3000 ml), washed with saturated sodium bicarbonate (2 x 400 ml) and brine (500 ml) and dried over sodium sulfate. After removal of solvent, 302 g of **15** was obtained as a brown oil.

D) 2-(4-(Bis-(3,4-difluoro-phenyl)-methyl)-Piperazine-1-carbonyl)-piperidine-1-carboxylic acid tert-butyl ester **16**

Pivaloyl chloride (53g, 0.44 mol, 1 eq.) was added dropwise over 1 hr at room temperature to a solution of (S)-(+)-1-(tert-butoxycarbonyl)-2-piperidinecarboxylic acid 7 (100 g, 0.44 mol) and triethylamine (89 g, 0.88 mol, 2.0 eq) in methylene chloride (1500 ml). After stirring another 2 hrs at room temperature, a solution of **15** (143 g, 0.44 mol) in methylene chloride (500 ml) was added over 2 hrs. The reaction mixture was stirred overnight at room temperature, then washed with NaOH (1N, 800 ml) and brine (2 x 500 ml) and dried over sodium sulfate. After removal of solvent, the crude product was purified by chromatography on silica gel (heptane/ethyl acetate/triethylamine = 50/50/1) to give 188g (80%) of pure 16 as a colorless oil.

E) (4-(Bis-(3,4-difluoro-phenyl)-methyl)piperazine-1-yl)-piperidin-2-yl-methanone **17**

Trifluoroacetic acid (700 ml, 9.1 mol) was added to a solution of **16** (187.8 g, 0.35 mol) in methylene chloride (2000 ml) over 2 hrs. at room temperature. After stirring 1 hr at room temperature, TLC showed complete reaction and solvent was removed under vacuum. The residue was redissolved in 6L methylene chloride, washed with 1N NaOH (2 x 600 ml) and brine (2 x 600 ml) and dried over sodium sulfate. After removal or solvent the crude product was purified on silica gel (methylene chloride/methanol/ammonium hyrdroxide = 12/1/0.5) to give 100 g of pure **17** as a colorless oil.

Mass Spec: M+1 m/z = 436 (API-ES, positive mode). Optical Rotation: [α]_{D} = -2.7 (c = 0.548 g/100 ml CHCl₃). HPLC (C18 column (50 mm)) Rt 4.007 mins. (99.7 %).

F) (4-(Bis-(3,4-difluoro-phenyl)-methyl)piperazine-1-yl)-piperidin-2-yl-methanone dihydrochloride, **17**

The compound **17** (5.2 g, 11.9 mmol) was dissolved in 20 ml of methylene chloride and diluted with 200 ml of diethyl ether. An anhydrous solution of 1.0 M HCl in diethyl ether (70 ml, 70 mmol) was added dropwise. A precipitate formed and the after stirring for 1.5 hours, the precipitate was collected and dried under vacuum at 50 °C to give the dihydrochloride salt, 6.03 g. HPLC (C18 column (150 mm)): Rt 4.971 mins. (100% pure).

Comparative Example 3

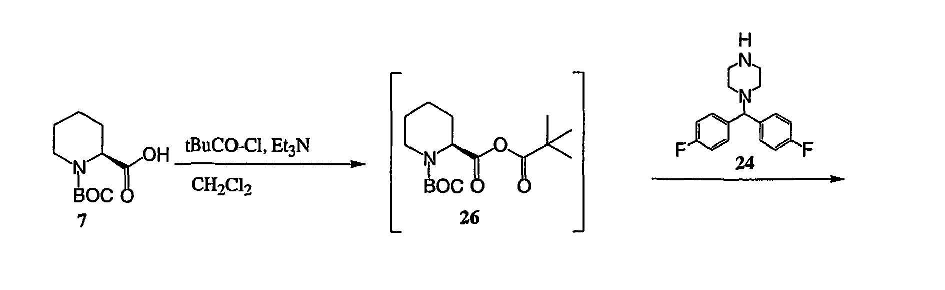

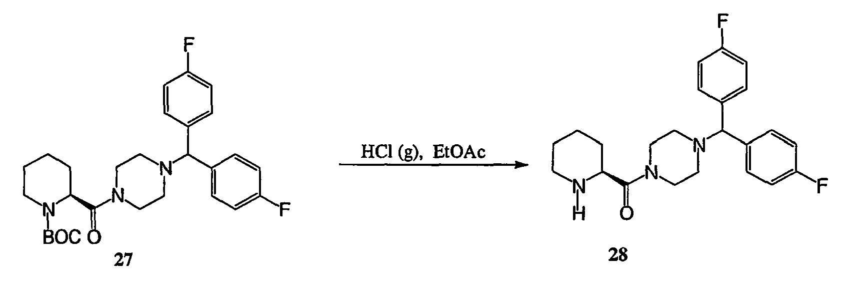

A) 2-{4-[Bis-(4-fluoro-phenyl)-methyl]-piperazine-1-carbonyl}-piperidine-1-carboxylic acid tert-butyl ester **27**

Pivaloyl chloride (4.3 ml, 34.9 mmol) was added dropwise over 15 mins. at room temperature to a solution of (S)-(+)-1-(tert-butoxycarbonyl)-2-piperidinecarboxylic acid (8 g, 34.89 mmol) and triethylamine (12.5 ml, 90 mmol) in methylene chloride (150 ml). After stirring for 1.5 hrs., a solution of 1-bis-(4,4'-difluoro-benzhydryl)piperazine (9.51 g, 33 mmol) in methylene chloride (100 ml) was added over 1.5 hrs. and the reaction stirred at room temperature overnight. The reaction was washed with 1N sodium hydroxide ( 2 x 100 ml) and brine and the organic layer dried over anhydrous sodium sulfate. After removal of solvent, the crude product was purified by chromatography (Silica gel) eluting with methylene chloride/ethyl acetate (7/3) to afford 16.5 g (quantitative yield) of the desired product.

B) (4-(Bis-(4-fluoro-phenyl)-methyl)piperazine-1-yl)-piperidin-2-yl-methanone dihydrochloride **28**

2-{4-[Bis-(4-fluoro-phenyl)-methyl]-piperazine-1-carbonyl}-piperidine-1-carboxylic acid tert-butyl ester (16.48 g, 33 mmol) was dissolved in ethyl acetate (250 ml) and cooled to 5 C. Anhydrous hydrogen chloride was bubbled into the solution and after 15 minutes a precipitate formed. The precipitate was filtered, washed with diethyl ether and dried under vacuum at 50 C to afford 15.7 g of the desired product

Mass Spec: M+1 m/z = 400.4 (ES, positive mode)
HPLC (C18 column (150 mm)) Rt 3.847 mins. (100%).

Example 4.

Neuroprotection Assay

The ventral mesencephalic region was dissected out of embryonic day 15 Sprague-Dawley rat embryos (Harlan), dissociated into single cell suspension by a combination of trypsinization and trituration (Costantini et al., Neurobiol Dis., pp. 97-106 (1998). Dissociated VM cells were plated into poly-L-ornithine-coated 96-well plates at a density of 85,000 cells/well in 100 uL of DMEM supplemented with 18% heat-inactivated horse serum, 0.24% glucose, 2 mM glutamine and 50 u/ml pernicillin/streptomycin and incubated in a 5% CO₂ incubator. After one day in culture (DIV1), the medium was replaced with 100 µL of a defined medium (DMEM supplemented with 1x N2 cocktail (Gibco-BRL), 0.12% glucose, 2 mM glutamine, and 50 units/ml penicillin/streptomycin) containing DMSO or various concentrations of the compounds of this invention. On DIV5, neuroexcitotoxic injury was induced by the addition of various concentrations of the glutamate receptor agonist NMDA (100-400 µM). Cultures were incubated with the neurotoxin for 20 hours and the effects of neurophilin compounds were assessed using high affinity ³H-dopamine uptake according to a procedure published by Park and Mytilineou [Brain Res., 599, pp. 83-97 (1992)].

The Table below shows the results of this assay for various compounds of this invention.

Table 1. Compound Activity.

| VRT | DAT | DRG |
|---|---|---|
| # | (VM) | neurite |
| | IC50 | outgrowth |
| | (nM) | |
| Comparative 28 | 12 | + |
| 9 | 145 | n.d. |
| 17 | 6 | n.d. |

It is expected that all compounds of this invention will show detectable activity in this assay.

While we have described a number of embodiments of this invention, it is apparent that our basic examples may be altered to provide other embodiments which utilize the compounds and methods of this invention. Therefore, it will be appreciated that the scope of this invention is to be defined by the appended claims rather than by the specific embodiments which have been represented by way of example.

## Claims

1. A compound having formula IA: 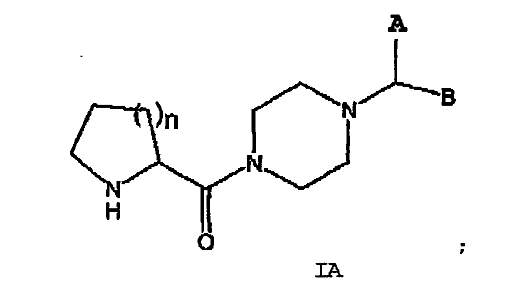 wherein:
n is 1 or 2;
A and B each is independently selected from phenyl, chlorophenyl, dichlorophenyl, fluorophenyl, or difluorophenyl provided that if n is 2 A, B are not simultaneously phenyl and that the compound having formula IA is not

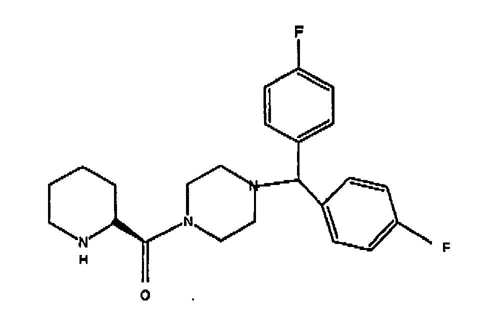

2. The compound according to claim 1, wherein said compound is selected from any one of compounds 9 or, 17.

3. A composition comprising a compound according to any one of claims 1 to 2 in an amount sufficient to stimulate nerve growth or prevent neurodegeneration; and a pharmaceutically acceptable carrier.

4. The composition according to claim 3, additionally comprising a neurotrophic factor.

5. The composition according to claim 4, wherein said neurotrophic factor is selected from nerve growth factor (NGF), insulin-like growth factor (IGF-1) and its active truncated derivatives such as gIGF-1 and Des(1-3)IGF-1, acidic and basic fibroblast growth factor (aFGF and bFGF, respectively), platelet-derived growth factors (PDGF), brain-derived neurotrophic factor (BDNF), ciliary neurotrophic factors (CNTF), glial cell line-derived neurotrophic factor (GDNF), neurotrophin-3 (NT-3) and neurotrophin 4/5 (NT-4/5).

6. The composition according to claim 3, wherein said composition is formulated for oral or parenteral administration to a patient.

7. The composition according to claim 4, wherein said composition is formulated for oral or parenteral administration to a patient.

8. The use of a compound according to claim 1 or 2 in the manufacture of a medicament for promoting neuronal repair or preventing neuronal damage in a patient comprising the step of administering to said patient an amount of the compound sufficient to promoting neuronal repair or preventing neuronal damage.

9. The use according to claim 8, comprising the additional step of administering to said patient a neurotrophic factor either as part of a multiple dosage from together with said compound or as a separate dosage form.

10. The use according to claim 9, wherein said neurotrophic factor is selected from nerve growth factor (NGF), insulin-like growth factor (IGF-1) and its active truncated derivatives such as gIGF-1 and Des (1-3)IGF-I, acidic and basic fibroblast growth factor (aFGF and bFGF, respectively), platelet-derived growth factors (PDGF), brain-derived neurotrophic factor (BDNF), ciliary neurotrophic factors (CNTF), glial cell line-derived neurotrophic factor (GDNF), neurotrophin-3 (NT-3)and neurotrophin 4/5 (NT-4/5).

11. The use according to claim 8, wherein said method is used to treat a patient suffering from a disease selected from trigeminal neuralgia, glosspharyngeal neuralgia, Bell's Palsy, myasthenia gravis, muscular dystrophy, muscle injury, progressive muscular atrophy, progressive bulbar inherited muscular atrophy, herniated, ruptured, or prolapsed invertebrae disk syndrome's, cervical spondylosis, plexus disorders, thoracic outlet destruction syndromes, peripheral neuropathies, such as those caused by lead, dapsone, ticks, or porphyria, other peripheral myelin disorders, Alzheimer's disease, Gullain-Barre syndrome, Parkinson's disease and other Parkinsonian disorders, ALS, Tourette's syndrome, multiple sclerosis, other central myelin disorders, stroke and ischemia associated with stroke, neural paropathy, other neural degenerative diseases, motor neuron diseases, sciatic injury, neuropathy associated with diabetes, spinal cord injuries, facial nerve injury and other trauma, chemotherapy- and other medication-induced neuropathies, Huntington's disease, and protein fibrillization diseases, such as Diffuse Lewy Body disease, Alzheimer's disease-Lewy Body variant, Familial British Dementia, and Frontotemporal Dementia.

12. The use according to claim 9, wherein said method is used to treat a patient suffering from a disease selected from trigeminal neuralgia, glosspharyngeal neuralgia, Bell's Palsy, myasthenia gravis, muscular dystrophy, muscle injury, progressive muscular atrophy, progressive bulbar inherited muscular atrophy, herniated, ruptured, or prolapsed invertebrae disk syndrome's, cervical spondylosis, plexus disorders, thoracic outlet destruction syndromes, peripheral neuropathies, such as those caused by lead, dapsone, ticks, or porphyria, other peripheral myelin disorders, Alzheimer's disease, Gullain-Barre syndrome, Parkinson's disease and other Parkinsonian disorders, ALS, Tourette's syndrome, multiple sclerosis, other central myelin disorders, stroke and ischemia associated with stroke, neural paropathy, other neural degenerative diseases, motor neuron diseases, sciatic injury, neuropathy associated with diabetes, spinal cord injuries, facial nerve injury and other trauma, chemotherapy- and other medication-induced neuropathies, Huntington's disease, and protein fibrillization diseases, such as Diffuse Lewy Body disease, Alzheimer's disease-Lewy Body variant, Familial British Dementia, and Frontotemporal Dementia.

13. A method for promoting neuronal repair or preventing neuronal damage in an ex vivo nerve cell, glial cell, chromafin cell or stem cell comprising the step of administering to said said cell an amount of a compound according to claim 1 or 2 sufficient to promoting neuronal repair or preventing neuronal damage.

## Patentansprüche

1. Verbindung mit der Formel IA: 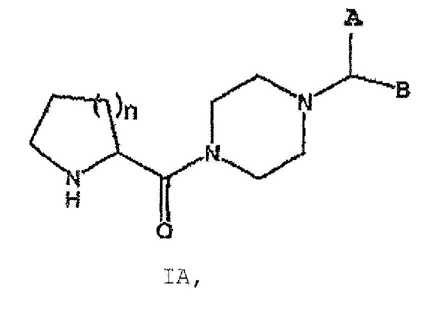 wobei:
- n 1 oder 2 bedeutet und
- A und B jeweils unabhängig voneinander aus Phenyl, Chlorphenyl, Dichlorphenyl, Fluorphenyl oder Difluorphenyl unter der Voraussetzung ausgewählt sind, dass, wenn n gleich 2 ist, A und B nicht gleichzeitig Phenyl bedeuten und die Verbindung mit der Formel IA nicht 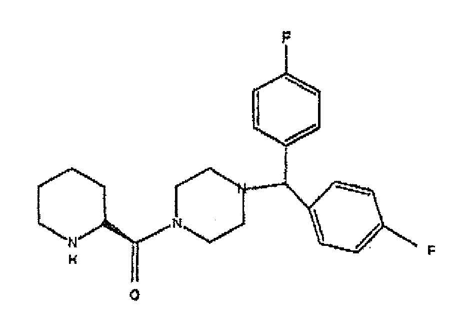 bedeutet.

2. Verbindung nach Anspruch 1, die aus Verbindung 9 oder 17 ausgewählt ist.

3. Zusammensetzung, die eine Verbindung nach Anspruch 1 oder 2 mit einem Anteil, der ausreicht, das Nervenwachstum zu stimulieren oder eine Neurodegeneration zu verhindern, und einen pharmazeutisch verträglichen Träger umfasst.

4. Zusammensetzung nach Anspruch 3, die zusätzlich einen neurotrophen Faktor umfasst.

5. Zusammensetzung nach Anspruch 4, wobei der neurotrophe Faktor aus Nervenwachstumsfaktor (NGF), Insulin-artigem Wachstumsfaktor (IGF-1) und dessen aktiven trunkierten Derivaten wie gIGF-1 und Des(1-3)IGF-I, saurem und basischem Fibroblastenwachstumsfaktor (aFGF bzw. bFGF), Plättchen-entstammenden Wachstumsfaktoren (PDGF), *brain-derived neurotrophic factor* (BDNF), ciliary neurotrophic factors (CNTF), *glial cell line-derived neurotrophic factor* (GDNF), Neurotrophin-3 (NT-3) und Neurotrophin 4/5 (NT-4/5) ausgewählt ist.

6. Zusammensetzung nach Anspruch 3, die für eine orale oder parenterale Verabreichung an einen Patienten formuliert ist.

7. Zusammensetzung nach Anspruch 4, die für eine orale oder parenterale Verabreichung an einen Patienten formuliert ist.

8. Verwendung einer Verbindung nach Anspruch 1 oder 2 zur Herstellung eines Arzneimittels für die Beschleunigung der Wiederherstellung von Neuronen oder die Verhinderung von Neuronenschäden bei einem Patienten, welche die Stufe der Verabreichung einer Menge der Verbindung, die ausreicht, um die Wiederherstellung von Neuronen zu beschleunigen oder Neuronenschäden zu verhindern, an diesen Patienten umfasst.

9. Verwendung nach Anspruch 8, welche die zusätzliche Stufe der Verabreichung eines neurotrophen Faktors entweder als Bestandteil einer Mehrfachdosierungsform zusammen mit der Verbindung oder als separate Dosierungsform an den Patienten umfasst.

10. Verwendung nach Anspruch 9, wobei der neurotrophe Faktor aus Nervenwachstumsfaktor (NGF), Insulin-artigem Wachstumsfaktor (NGF-1) und dessen aktiven trunkierten Derivaten wie gIGF-1 und Des(1-3)IGF-I, saurem und basischem Fibroblastenwachstumsfaktor (aFGF bzw. bFGF), Plättchen-entstammenden Wachstumsfaktoren (PDGF), *brain-derived neurotrophic factor* (BDNF), ciliary neurotrophic factors (CNTF), *glial cell line-derived neurotrophic factor* (GDNF), Neurotrophin-3 (NT-3) und Neurotrophin 4/5 (NT-4/5) ausgewählt ist.

11. Verwendung nach Anspruch 8, wobei das Verfahren angewendet wird, um einen Patienten zu behandeln, der an einer Krankheit leidet, die aus Trigeminusneuralgie, Glossopharyngeusneuralgie, Bell-Phänomen, Myasthenia gravis, Muskeldystrophie, Muskelverletzung, progressiver Muskelatrophie, progressiver bulbärer hereditärer Muskelatrophie, dem Syndrom einer gebrochenen, gerissenen oder vorgefallenen Bandscheibe, Halsspondylose, Plexuskrankheiten, Thoracic-outlet-destruction-Syndrom, peripheren Neuropathien wie denjenigen, die von Blei, Dapson, Zecken oder Porphyrie verursacht werden, anderen peripheren Myelinerkrankungen, Alzheimer-Krankheit, Guillain-Barré-Syndrom, Parkinson-Syndrom und anderen Parkinson-Krankheiten, ALS, Tourette-Syndrom, Multiple Sklerose, anderen zentralen Myelinerkrankungen, Schlaganfall und mit einem Schlaganfall einhergehender Ischämie, neuraler Paropathie, anderen degenerativen Nervenerkrankungen, Motor-Neuron-Diseases, Hüftverletzung, mit Diabetes einhergehender Neuropathie, Rückenmarksverletzungen, Gesichtsnervenverletzung und anderen Traumata, von einer Chemotherapie und anderen Medikationen ausgelösten Neuropathien, Chorea-Huntington und Proteinfibrillierungskrankheiten wie diffuser Lewy-Körperdemenz, Alzheimer-Krankheit (Lewy-Körperdemenz-Variante), Familial British Dementia und frontotemporaler Demenz ausgewählt ist.

12. Verwendung nach Anspruch 9, wobei das Verfahren angewendet wird, um einen Patienten zu behandeln, der an einer Krankheit leidet, die aus Trigeminusneuralgie, Glossopharyngeusneuralgie, Bell-Phänomen, Myasthenia gravis, Muskeldystrophie, Muskelverletzung, progressiver Muskelatrophie, progressiver bulbärer hereditärer Muskelatrophie, dem Syndrom einer gebrochenen, gerissenen oder vorgefallenen Bandscheibe, Halsspondylose, Plexuskrankheiten, Thoracic-outlet-destruction-Syndrom, peripheren Neuropathien wie denjenigen, die von Blei, Dapson, Zecken oder Porphyrie verursacht werden, anderen peripheren Myelinerkrankungen, Alzheimer-Krankheit, Guillain-Barré-Syndrom, Parkinson-Syndrom und anderen Parkinson-Krankheiten, ALS, Tourette-Syndrom, Multiple Sklerose, anderen zentralen Myelinerkrankungen, Schlaganfall und mit einem Schlaganfall einhergehender Ischämie, neuraler Paropathie, anderen degenerativen Nervenerkrankungen, Motor-Neuron-Diseases, Hüftverletzung, mit Diabetes einhergehender Neuropathie, Rückenmarksverletzungen, Gesichtsnervenverletzung und anderen Traumata, von einer Chemotherapie und anderen Medikationen ausgelösten Neuropathien, Chorea-Huntington und Proteinfibrillierungskrankheiten wie diffuser Lewy-Körperdemenz, Alzheimer-Krankheit (Lewy-Körperdemenz-Variante), Familial British Dementia und frontotemporaler Demenz ausgewählt ist.

13. Verfahren zur Beschleunigung der Wiederherstellung von Neuronen oder zur Verhinderung von Neuronenschäden in einer *ex-vivo*-Nervenzelle, Gliazelle, chromaffinen Zelle oder Stammzelle, das die Stufe der Verabreichung einer Menge einer Verbindung nach Anspruch 1 oder 2, die ausreicht, die Wiederherstellung von Neuronen zu beschleunigen oder eine Neuronenschädigung zu verhindern, an diese Zelle umfasst.

## Revendications

1. Composition de formule IA 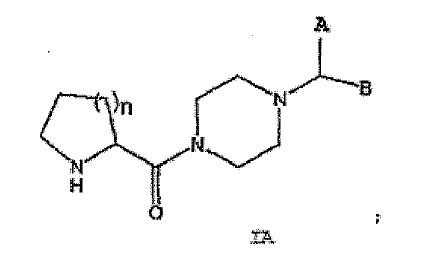 dans laquelle
n vaut 1 ou 2 ;
A et B sont chacun choisis indépendamment parmi phényle, chlorophényle, dichlorophényle, fluorophényle, ou difluorophényle, avec la condition que, si n vaut 2, A, B ne représentent pas simultanément phényle, et que le composé de formule IA n'est pas

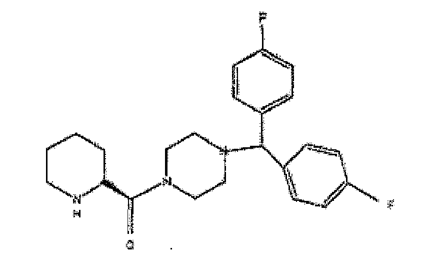

2. Composé selon la revendication 1, dans lequel ledit composé est choisi parmi l'un quelconque des composés 9 et 17.

3. Composition comprenant un composé selon l'une quelconque des revendications 1 à 2, en quantité suffisante pour stimuler la croissance neurale ou éviter une neurodégénérescence ; et un support pharmaceutiquement acceptable.

4. Composition selon la revendication 3, comprenant de plus un facteur neurotrophique.

5. Composition selon la revendication 4, dans laquelle ledit facteur neurotrophique est choisi parmi les facteur de croissance neurale (NGF), le facteur de croissance semblable à l'insuline (IGF-1) et ses dérivés tronqués actifs tels que gIGF-1 et Des(1-3)IGF-1, le facteur de croissance des fibroblastes, acide et basique (aFGF et hFGF, respectivement), les facteurs de croissance dérivés de plaquettes (PDGF), le facteur neurotrophique dérivé du cerveau (BDNF), des facteurs neurotrophiques ciliaires (CNTF), le facteur neurotrophique dérivé de lignées de cellules gliales (GDNF), la neurotrophine-3 (NT-3) et la neurotrophine 4/5 (NT-4/5).

6. Composition selon la revendication 3, dans laquelle ladite composition est formulée pour une administration orale ou parentérale, à un patient.

7. Composition selon la revendication 4, dans laquelle ladite composition est formulée pour une administration orale ou parentérale, à un patient.

8. Utilisation d'un composé selon la revendication 1 ou 2, dans la fabrication d'un médicament pour favoriser une réparation neuronale ou empêcher une lésion neuronale chez un patient, comprenant l'étape consistant à administrer audit patient une quantité du composé suffisante pour favoriser une réparation neuronale ou empêcher une lésion neuronale.

9. Utilisation selon la revendication 8, comprenant l'étape supplémentaire consistant à administrer audit patient un facteur neurotrophique, soit en tant que partie d'une forme pharmaceutique multiple, conjointement avec ledit composé, soit en tant que forme pharmaceutique séparée.

10. Utilisation selon la revendication 9, dans laquelle ledit facteur neurotrophique est choisi parmi le facteur de croissance neurale (NGF), le facteur de croissance semblable à l'insuline (IGF-1) et ses dérivés tronqués actifs tels que gIGF-1 et Des(I-3)IGF-1, le facteur de croissance des fibroblastes, acide et basique (aFGF et hFGF, respectivement), les facteurs de croissance dérivés de plaquettes (PDGF), le facteur neurotrophique dérivé du cerveau (BDNF), des facteurs neurotrophiques ciliaires (CNTF), le facteur neurotrophique dérivé de lignées de cellules gliales (GDNF), la neurotrophine-3 (NT-3) et la neurotrophine 4/5 (NT-4/5).

11. Utilisation selon la revendication 8, dans laquelle ledit procédé est utilisé pour traiter un patient souffrant d'une affection choisie parmi névralgie du trijumeau, névralgie glossopharyngée, paralysie de Bell, myasthénie gravis, dystrophie musculaire, lésion musculaire, myopathie primitive progressive, paralysie bulbaire progressive héréditaire, syndromes de hernie discale ou prolapsus de disque intervertébral, spondylarthrose cervicale, troubles du plexus, syndromes de destruction de la traversée thorocobrachiale, neuropathies périphériques, telles que celles causées par le plomb, le dapsone, les tiques ou une porphyrie, autres troubles de la myéline périphérique, maladie d'Alzheimer, syndrome de Gullain-Barre, maladie de Parkinson et autres troubles parkinsoniens, SLA, syndrome de Tourette, sclérose multiple, autres troubles de la myéline centrale, accident cérébrovasculaire et ischémie associée à un accident cérébrovasculaire, paropathie neurale, autres affections neurales dégénératives, affections neuronales motrices, lésion sciatique, neuropathie associée aux diabètes, lésions de la moelle épinière, lésion des nerfs faciaux et autres trauma, neuropathies induites par chimiothérapie et d'autres médications, affection de Huntington, et affections de fibrillisation protéique, telles que l'affection à corps de Lewy diffus, la variante de la maladie d'Alzheimer à corps de Lewy, la démence britannique familiale et la démence frontotemporale.

12. Utilisation selon la revendication 9, dans laquelle ledit procédé est utilisé pour traiter un patient soufrant d'une affection choisie parmi névralgie du trijumeau, névralgie glossopharyngée, paralysie de Bell, myasthénie gravis, dystrophie musculaire, lésion musculaire, myopathie primitive progressive, paralysie bulbaire progressive héréditaire, syndromes de hernie discale ou prolapsus de disque intervertébral, spondylarthrose cervicale, troubles du plexus, syndromes de destruction de la traversée thorocobrachiale, neuropathies périphériques, telles que celles causées par le plomb, le dapsone, les tiques ou une porphyrie, autres troubles de la myéline périphérique, maladie d'Alzheimer, syndrome de Gullain-Barre, maladie de Parkinson et autres troubles parkinsoniens, SLA, syndrome de Tourette, sclérose multiple, autres troubles de la myéline centrale, accident cérébrovasculaire et ischémie associée à un accident cérébrovasculaire, paropathie neurale, autres affections neurales dégénératives, affections neuronales motrices, lésion sciatique, neuropathie associée aux diabètes, lésions de la moelle épinière, lésion des nerfs faciaux et autres trauma, neuropathies induites par chimiothérapie et d'autres médications, affection de Huntington, et affections de fibrillisation protéique, telles que l'affection à corps de Lewy diffus, la variante de la maladie d'Alzheimer à corps de Lewy, la démence britannique familiale et la démence frontotemporale.

13. Procédé pour promouvoir une réparation neuronale ou empêcher une lésion neuronale dans une cellule neurale, une cellule gliale, une cellule chromaffine ou une cellule souche *ex vivo*, comprenant l'étape consistant à administrer à ladite cellule une quantité d'un composé selon la revendication 1 ou 2, suffisante pour favoriser une réparation neuronale ou empêcher une lésion neuronale.
